# EUROPEAN PATENT SPECIFICATION

(11) **EP 3 924 068 B1**
(45) Date of publication and mention of the grant of the patent: **23.07.2025**
(21) Application number: 20710721.0
(22) Date of filing: 14.02.2020
(51) Int. Cl.: A63B 23/16, A63B 21/02, A63B 21/04, A63B 71/06, A63B 23/035, A61B 5/11, A63B 21/05, A63B 21/002

(54) **HANDGRIP RELAXOMETER SYSTEM**
HANDGRIFF-RELAXOMETERSYSTEM
SYSTÈME DE RELAXOMÈTRE À POIGNÉE

(30) Priority: 14.02.2019 US 201962805750 P
(43) Date of publication of application: 22.12.2021
(73) Proprietor: The United States of America, as represented by The Secretary, Department of Health and Human Services, Bethesda, Maryland 20892-7660 (US)
(72) Inventor: MANKODI, Ami, Bethesda, Maryland 20892-7660 (US); GRAVUNDER, Andrew, Bethesda, Maryland 20892-7660 (US); BULEA, Thomas, Bethesda, Maryland 20892-7660 (US); DAMIANO, Diane, Bethesda, Maryland 20892-7660 (US)
(74) Representative: Sedlacek, Anton
(86) International application number: PCT/US2020/018273
(87) International publication number: WO 2020/168179

(56) References cited:
- WO-A1-2014/074809
- US-A- 5 157 970
- US-A- 5 317 916
- US-A1- 2006 035 762
- US-A1- 2016 120 446

## Description

### FIELD

The present disclosure relates generally to apparatuses and systems for identifying symptoms of myotonic dystrophy. More specifically, the present disclosure generally relates to devices and systems operable for measuring muscle grip and/or relaxation related to myotonic dystrophy. Related devices are disclosed, for example, in US 5,157,970 A, WO 2014/074809 A1, US 2006/035762 A1, US 2016/120446 A1 and US 5,317,916 A.

### BACKGROUND

Myotonic dystrophy is the most common genetically inherited muscle disease in adults. Myotonic dystrophy is a genetic disorder that causes progressive muscle weakness. One primary symptom of myotonic dystrophy is handgrip myotonia, which is characterized by the inability or delayed relaxation of muscles following vigorous effort. Current methods for identifying biomarkers with respect to myotonic dystrophy generally involve measuring grip strength with a handheld dynamometer that requires a specific position. These handheld dynamometers are not capable of measuring full grip force and cannot measure continuous force during relaxation. Additionally, these devices are costly, obtrusive, and are not suitable for weaker muscles and/or mild cases of myotonia.

### BRIEF DESCRIPTION OF THE DRAWINGS

According to the invention, the problem posed is solved by the handgrip device of claim 1, handgrip system of claim 5 and the method of handgrip monitoring of claim 8. A better understanding of the features and advantages of the present disclosure will be obtained by reference to the following detailed description that sets forth illustrative examples, in which the principles of the disclosure are utilized, and the accompanying drawings of which:
FIG. 1 is a diagrammatic view of an handgrip myotonia detection system, according to the present disclosure;
FIG. 2A is an isometric view of a handgrip myotonia detection apparatus, according to a comparative example;
FIG. 2B is an isometric view of a handgrip myotonia detection apparatus having two load cells, according to a comparative example;
FIG. 3 is an isometric view of a handgrip myotonia detection apparatus having a user operably engaged therewith, according to a comparative example;
FIG. 4 is a graphical view of a handgrip detection measurement of the handgrip myotonia detection system of FIG. 1;
FIG. 5 is an isometric view of a handgrip myotonia detection apparatus, according to the present disclosure.
FIG. 6 is an left isometric view of the handgrip myotonia detection apparatus of FIG. 5, according to the present disclosure;
FIG. 7 is an isometric view of a handgrip myotonia detection apparatus of FIG. 5 having a user operably engaged therewith, according to the present disclosure;
FIG. 8 is a graphical view of a handgrip detection measurement of the handgrip myotonia detection system of FIG. 5, according to the present disclosure; and
FIG. 9 is a method of measuring handgrip myotonia, according to of the present disclosure.

### DETAILED DESCRIPTION

Examples and various features and advantageous details thereof are explained more fully with reference to the exemplary, and therefore non-limiting, examples illustrated in the accompanying drawings and detailed in the following description. Descriptions of known starting materials and processes can be omitted so as not to unnecessarily obscure the disclosure in detail.

The present disclosure is drawn to handgrip myotonia devices and related systems operable to measure, track, and/or display both muscle contraction (e.g. grip force) and/or muscle relaxation following muscle contraction. The handgrip myotonia system can include an electronic device communicatively coupled with a handgrip myotonia device. The electronic device can be a computer, including but not limited to, a laptop having a processor operable to implement a software suite installed in memory. The electronic device can generate a cue to request a user to contract their finger muscles (e.g. grip force) on a handgrip myotonia device followed by a second cue to relax their finger muscles. The software can receive force measurements from the handgrip myotonia device and/or generate a display having the received force measurements over time correlated with the cue requesting contraction and relaxation of the finger muscles. The generated display can be a digital display of the electronic device, a print out, and/or display on a secondary electronic device. In at least one instance, the software suite can be a graphical user interface (GUI) based software suite.

The electronic device can be wired and/or wirelessly communicatively coupled with the handgrip myotonia device, thereby allowing receipt of one or more force measurements from the handgrip myotonia device.

The handgrip myotonia device can be operable to measure both muscle contraction and muscle relaxation over time and/or communicate with the electronic device and the software suite installed thereon. The handgrip myotonia device can include one or more force transducers operable to receive and/or measure substantially continuously a grip force provided by a user upon receiving the cue. After the second cue, the one or more force transducers can receive and measure substantially continuous a relaxation force provided by the user.

The grip force and the relaxation force can be tracked and/or plotted by the software suite over time, allowing an operator to determine the ability of the user to generate a grip force and the user's muscles ability to relax following generation of the grip force. Tracking the grip force and the relaxation force with respect to time can provide an operator and/or a medical professional critical information regarding whether the user may or may not have symptoms of myotonic dystrophy.

The handgrip myotonia device can be operable to isolate specific muscle movements of the hand, specifically the fingers, while also being operably arranged to minimize wrist, elbow, and shoulder muscle interaction with the measurements. The isolation of finger muscles can provide a more detailed measurement of a user's grip force and ability to relax muscles following contraction. The identification of myotonic dystrophy can include characterizing, measuring, and/or quantifying myotonia in assessing therapeutic response in one or more clinical trials.

In at least one instance, the handgrip myotonia device can be a hand clasp relaxometer. The handgrip myotonia device can include a palm rest operable to have a user's palm adjacently engaged and a grip bar operable to adjacently engage with a user's fingers. One or more load cells can be disposed between the palm rest and/or the grip bar. The one or more load cells can be operable to measure a compression force as a user's finger muscles contract (e.g. grip strength) and compress the grip bar toward the palm rest, thereby compressing the one or more load cells. During muscle relaxation, the one or more load cells can measure a relaxation force as the grip bar returns to a nominal position. In user's experiencing myotonic dystrophy, an inability to relax the hand muscles will be noticeable based on the one or more load cells detection of the grip bar returning to the nominal position. Alternatively, the grip bar may remain static or stationary as the one or more load cells measure the grip force and/or relaxation force as the user releases the grip bar.

In another instance, the handgrip myotonia device can be a myotonia relaxation actuator having a frame and a palm device mounted thereto. The palm device can be movably coupled with the frame, thereby allowing vertical and/or horizontal position adjustments of the palm device along the frame. In at least one instance, the frame and the palm device can have a tongue and groove arrangement to allow infinite positioning options, and thus micro-adjustment, of the palm device relative to the frame.

The palm device can include a palm rest and a finger grip lever. The finger grip level can be pivotally coupled with the palm rest, thereby allowing the software suite to measure a grip force and a relaxation force. A user can pivot the finger grip lever upon muscle contraction, and the finger grip lever can return to a nominal position upon muscle relaxation. In at least one instance, the palm device can include a servomotor received therein and be operable to detect the impedance and/or torque profile of the user's muscle grip and muscle relaxation. User muscle relaxation can generate a muscle relaxation profile measured as servomotor torque output. The servomotor can be operable to minimize interaction force with a user's grip force and/or relaxation force, via the closed loop feedback control.

The handgrip myotonia device and system can be operable to measure a user's muscle contraction and relaxation in any number of positions and/or orientations while simultaneously being cost effective and mobile. The handgrip myotonia device can allow a user's to measure grip strength and/or muscle relaxation in a position most comfortable for the user, thereby providing more accurate results.

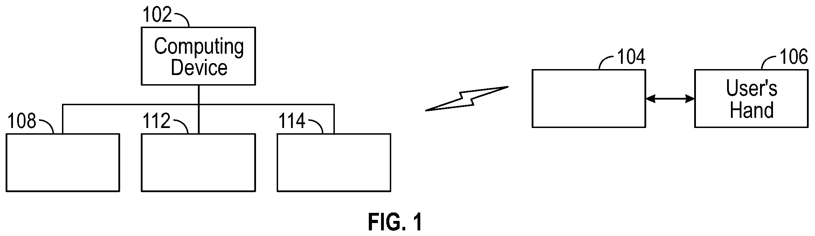

FIG. 1 illustrates a handgrip myotonia system according to at least one instance of the present disclosure. The handgrip myotonia system 100 can include an electronic device 102 and a handgrip myotonia device 104 The handgrip myotonia device 104 can be communicatively coupled with the electronic device 102 via wired and/or wireless communications. The handgrip myotonia device 104 can be operable to engage with a user 106 to measure muscle contraction force and/or relaxation force of a user's hand. The measured muscle contraction and/or relaxation data can be communicated to the electronic device 102 and a software suite 108 installed therein.

The software suite 108 can be installed on one or more memory devices 112 and executed by one or more processors 114 of the electronic device 102 The software suite 108 can generate a cue requesting a user 106 contract muscles and generate a second cue requesting a user relax muscles, while simultaneously receiving force data feedback from the handgrip myotonia device 104. The software suite 108 can generate a display of force generated and relaxation force over time and synchronized with the generated cues.

In at least one instance, the cue and second cue can be audio signals generated by the electronic device 102. The electronic device and/or software suite 108 can be operable to generate a display overlaying the audio signals with the measured contraction force and relaxation force.

The software suite 108 can generate a display 116 of the measured data for use with any medium including, but not limited to, digital displays (local displays and remote displays), and/or printed displays. In at least one instance, the display 116 can be a local display of the electronic device 102. In other instances, the display 116 can be a remote display located some distance away from the electronic device 102. The remote display can be a display within the same room, or a remote location. The remote display can allow an operator to measure the grip force and/or relaxation while communicating the results to one or more medical professionals.

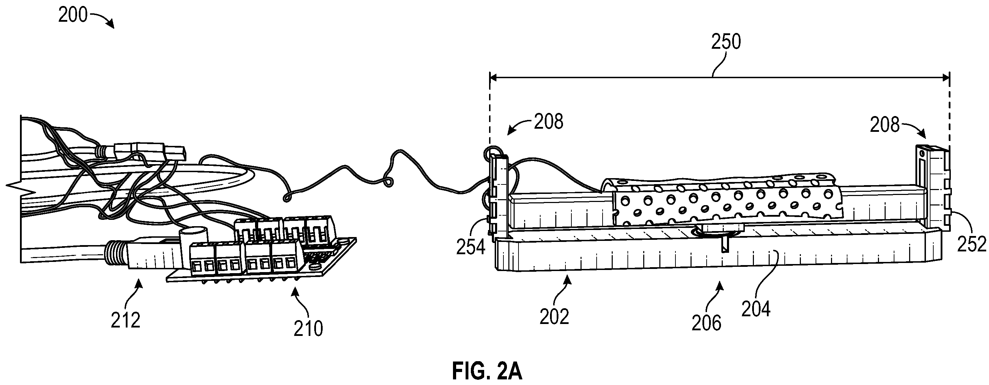

FIG. 2A illustrates an isometric view of a handgrip myotonia device according to a comparative example. The handgrip myotonia device 200 can be operable to measure both a muscle contraction (e.g. grip force) and a muscle relaxation of a user, thereby providing useful information regarding potential symptoms of myotonic dystrophy.

The handgrip myotonia device 200 can include a palm rest 202 and a grip bar 204. The palm rest 202 can be operable to adjacently engage with a user's palm during operation while the grip bar 204 can be operable to engage with fingers of a user during operation. The palm rest 202 can be in sliding engagement with the grip bar 204, and the grip bar 204 can be operable to slide relative to the palm rest 202. The palm rest 202 and the grip bar 204 can have one or more force transducers 206 disposed therebetween.

In at least one instance, the grip bar 204 can be statically coupled with the palm rest 202, but formed of a material capable of deformation relative to the palm rest 202. The grip bar 204 can have a strain gauge coupled therewith operable to allow a deformation measurement, and thus a grip strength measurement to be determined.

The one or more force transducers 206 can be operable to measure compression force when a user's muscle contraction pushes the grip bar 204 toward the palm rest 202. The one or more force transducers 206 can further be operable to measure relaxation force as the grip bar 204 returns to a nominal position relative to the palm rest 202 as a user's muscles relax following contraction.

The one or more force transducers 206 can be any known force measurement device operable to be disposed between the palm rest 202 and the grip bar 204. In at least one instance, the one or more force transducers 206 can be one or more load cells. The handgrip myotonia device 200 can be modularly arranged, thereby allowing addition and/or removal of force transducers 206 based on the necessary arrangement. An increased quantity of force transducers 206 can be implemented to more precisely measure weak muscle contraction and/or very strong muscle contractions.

The palm rest 202 and the grip bar 204 can be in sliding engagement with one another. The palm rest 202 and the grip bar 204 can be coupled via linear bearings 208, thereby allowing sliding engagement. The linear bearings 208 can be operable to transfer grip force from the user's fingers to the one or more force transducers 206. The linear bearings 208 can include a tongue and groove arrangement with at least a portion of the palm rest 202 and/or the grip bar 204 received with other. In other instances, the palm rest 202 and the grip bar 204 can be coupled via linear actuators, or any other mechanism allowing linear movement between the palm rest 202 and the grip bar 204.

In some instances, the linear bearings 208 can be biasing elements (e.g. springs) operable to bias the grip bar 204 towards a nominal position. In these instances, the biasing element is calibrated to insure the biasing force (return force) is less than the relaxation force of a user's hand. In other instances, the biasing elements may be placed in parallel or alternatively, in series with the bearings 208 and the force transducers 206. The calibration of the biasing element is essential to prevent the biasing element from assisting muscle relaxation of a user's hand.

The handgrip myotonia device 200 can be coupled with a force processing circuit 210. The force processing circuit 210 can be operable to process data received from the one or more force transducers 206 to a format appropriate for the software suite. The handgrip myotonia device 200 can further include a data connection 212 operable to communicate the processed data to the electronic device and/or software suite.

In at least one instance, the data connection 212 can be a universal serial bus (USB) connection. In other instances, the data connection 212 can be a network connection (e.g. RJ-45), wireless connection (WiFi, Bluetooth, etc.), or any other data connection.

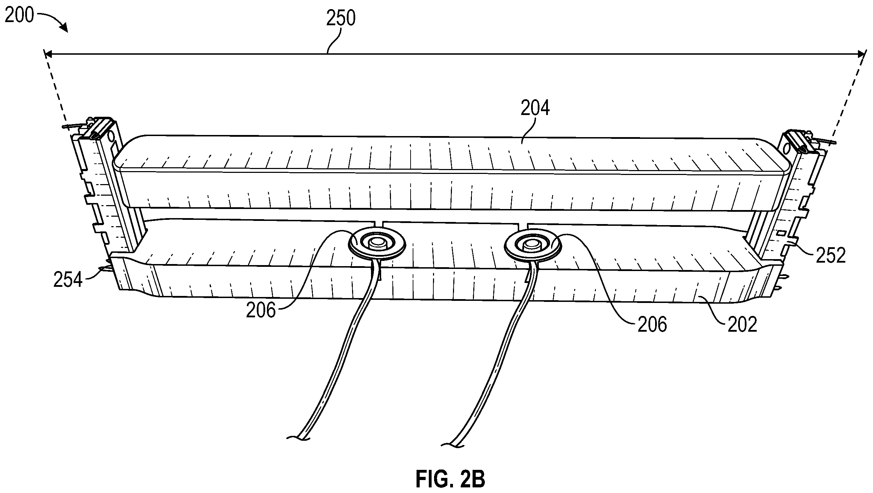

The handgrip myotonia device 200 can include a plurality of force transducers 206 as illustrated with respect to FIG. 2B. The plurality of force transducers 206 can be substantially equally spaced along the longitudinal length 250 of the hand myotonia device 200. In some instances, the plurality of force transducers 206 can be substantially equally spaced apart relative to a centerline of the longitudinal length 250. In other instances, the plurality of force transducers 206 can be substantially equally spaced relative to a proximal end 252 and a distal end 254, respectively.

As can be appreciated in FIG. 2B, the handgrip myotonia device 200 can incorporate two force transducers 206 substantially evenly spaced long the longitudinal length 250 of the handgrip myotonia device 200. The two force transducers 206 can be operable to prevent rotation of palm rest 202 relative to the grip bar 204 during muscle contraction and/or relaxation by a user. The inhibiting of rotation between the palm rest 202 and grip bar 204 can prevent distortion of force assessments, thereby providing accurate assessments of grip force for each user.

While FIG. 2A and FIG. 2B illustrates a handgrip myotonia device 200 having one force transducer 206 and two force transducers 206, respectively disposed therein, they may include any number force transducers as necessary to measure appropriate muscle contraction and/or relaxation along the palm rest 202 and/or the grip bar 204.

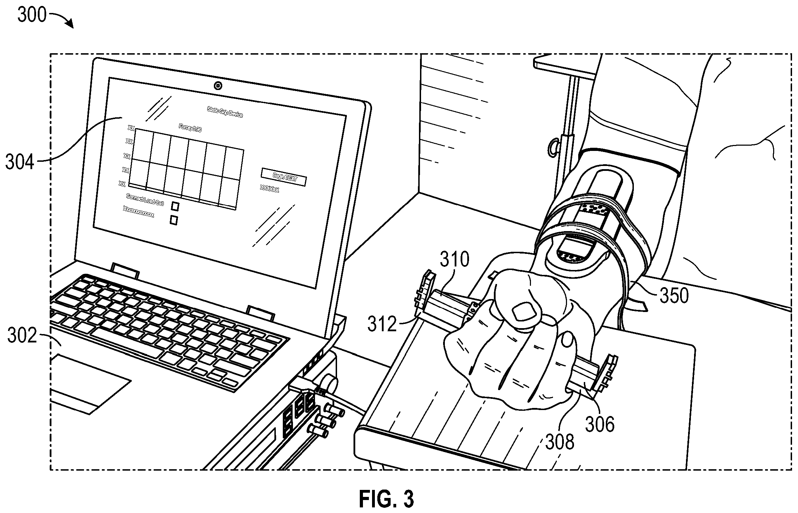

FIG. 3 illustrates a handgrip myotonia system including a handgrip myotonia device in operably engaged with a user. The handgrip myotonia system 300 can include an electronic device 302 having a software suite 304 installed thereon. A handgrip myotonia device 306 can be communicatively coupled with the electronic device 302.

The handgrip myotonia device 306 can have a user' s hand 350 operably engaged therewith. The handgrip myotonia device 306 can have a palm rest 308 adjacently engaged with the palm of the user's hand 350 and a grip bar 310 adjacently engaged with the fingers of the user's hand 350.

During operation, the software suite 304 can generate a cue (e.g. audio, visual, and/or haptic) to the user requesting the user contract the finger muscles in the hand 350, thereby requesting the compression of the one or more force transducers 312. As user contracts finger muscles (e.g. grip strength) the grip bar 310 can be linearly displaced toward the palm rest 308, thereby compressing the one or more force transducers 312. The compression of the one or more force transducers 312 can produce a force measurement received, recorded, and/or displayed by the software suite 304 on the electronic device 302.

The software suite can then generate a second cue (e.g. audio, visual, and/or haptic) to the user requesting the relaxation of finger muscles in the hand 350, thereby allowing the grip bar 310 to return to a nominal position relative to the palm rest 308 in embodiments having a dynamic grip bar. The one or more force transducers 312 can track the relaxation of the muscles and communicate the measurements to the software suite 304 for recording and/or display. The software suite 304 can overlay and/or time synchronize the cue, second cue, compression force and/or the relaxation force on a single plot.

The cue and second cue generated by the software suite 304 and/or the electronic device 302 can be an audio signal, visual signal, haptic signal, and/or any other signal operable to request or signal the user to contract and/or relax finger muscles in the hand 350. The cue and/or the second cue can be synchronized (e.g. by the software suite) with handgrip myotonia device measurement, such that the cue and/or the second cue can be recorded as a time series with substantially the same sampling rate as any other measurements from the handgrip myotonia device.

As can be appreciated in FIG. 3, the user's hand 350 can be restrained against a rigid surface in an effort to isolate finger muscle contraction and relaxation while eliminating and/or minimizing adjacent muscle groups, such as wrist, forearm, elbow and/or shoulder.

While FIG. 3 illustrates the user's hand 350 in a substantially palm up arrangement, the handgrip myotonia device(s) and/or system(s) of the present disclosure can be implemented in any arrangement comfortable for the user. Further, while FIG. 3 illustrates the user's hand 350 right- hand, the handgrip myotonia device(s) and/or system(s) are operably arranged to be ambidextrous to be implemented with a user's right-hand or left hand.

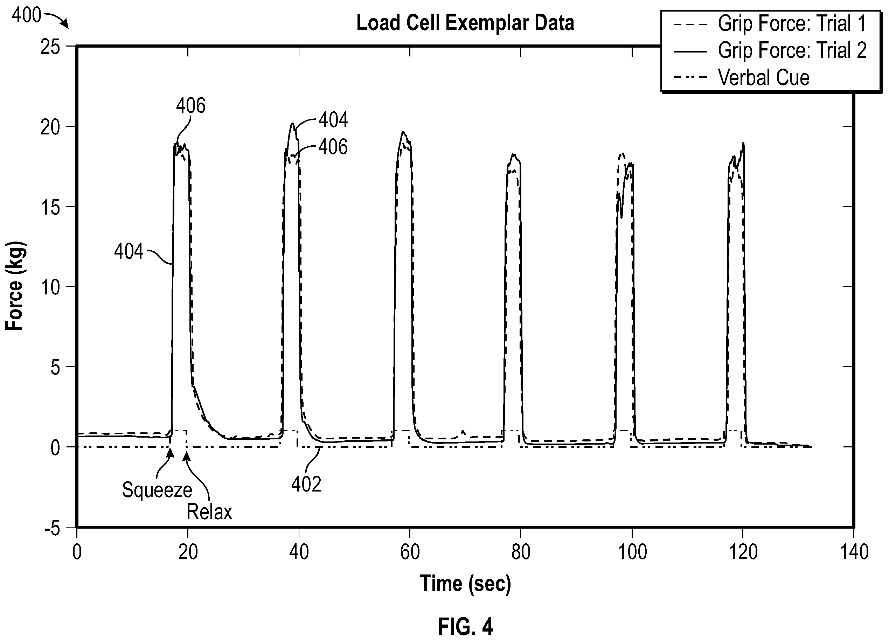

FIG. 4 illustrates a force chart generated by the handgrip myotonia system as described. The force chart 400 displays two data sets recorded by the handgrip myotonia device, along with the cues (e.g. cue and second cue) generated by the software suite of the handgrip myotonia system.

The force chart 400 can be a plot of generate force (e.g. grip strength) over time. The cue 402 can be overlaid on the force chart 400, thereby allowing representation of reaction time of both the user and/or the user's muscles. The measured force 404, 406 can be plotted as independent trials representative of multiple operations of the handgrip myotonia device. The measured force 404, 406 can show the user's generated force in both a muscle contraction and a muscle relaxation situation by overlayment of the cue. The force chart 400 can detail the user's ability to contract muscles upon receipt of the cue and similarly the ability of the user's muscles to relax upon receipt of the second cue after contraction. Thus, a delayed muscle relaxation can be identified within the force chart 400 and/or the handgrip myotonia system, thereby allowing medical professionals to potentially identify myotonic dystrophy. The identification of myotonic dystrophy can include characterizing, measuring, and/or quantifying myotonia in assessing therapeutic response in one or more clinical trials.

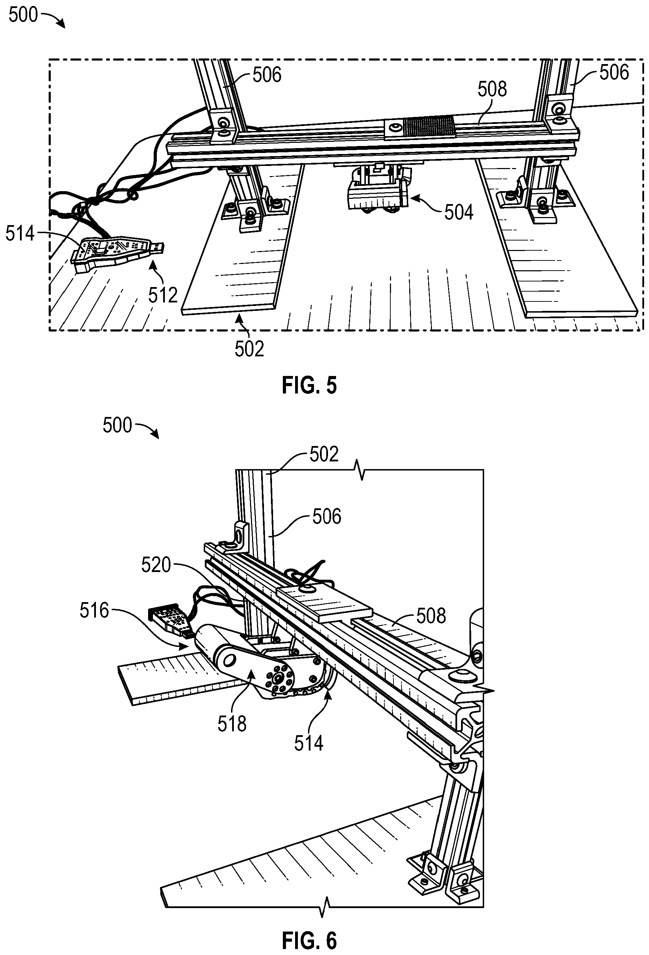

FIG. 5 illustrates a handgrip myotonia device being a myotonia relaxation actuator, according to the present disclosure. The handgrip myotonia device 500 can include a myotonia relaxation actuator. The handgrip myotonia device 500 can include a frame 502 and a palm device 504. The palm device 504 can be in sliding engagement with the frame 502, thereby allowing the palm device to be positioned at any desired position on the frame 502. Further, the frame 502 can have a vertical frame 506 and a horizontal frame 508. The horizontal frame 508 can be in sliding engagement with the vertical frame 506, thereby allowing the vertical position of the horizontal frame 508 to be independently adjusted. While FIG. 5 illustrates the palm device 504 moveably coupled with the horizontal frame 508, it is within the scope of this disclosure to implement the palm device 504 with the horizontal frame 508, the vertical frame 506, and/or a frame member disposed at any angle between horizontal and vertical, thereby allowing the handgrip myotonia device 500 to be positioned at the most comfortable position for a user.

The handgrip myotonia device 500 can be communicatively coupled with an electronic device (as described in FIG. 1) by data connection 512 and have a force processing circuit 514.

FIG. 6 illustrates a side isometric view of the handgrip myotonia device of FIG. 5. The palm device 504 can include a palm rest 514 and a finger grip lever 516. The finger grip lever 516 can be pivotally coupled with the palm rest 514 and operable to pivot as a user's finger muscles contract. The palm device 504 can have an actuator 518 disposed between the palm rest 514 and the finger grip lever 516. The actuator 518 may further include a sensor or force transducer to detect and/or measure force applied to pivot the finger grip lever 516 relative to the palm rest 514. In at least one instance, the actuator 518 can be a servomotor.

The actuator 518 can be coupled with an impedance feedback control loop allowing the actuator 518 to detect the force applied by a user's muscle contraction and match the measured torque to a net zero torque.

The palm device 504 can also include a position sensor 520 coupled with the palm rest 514 and the finger grip lever 516 and operable to determine a position of the finger grip lever 516 relative to the palm device 504. The position sensor 520 can provide additional information about a user's range of motion, while simultaneously allowing the software suite to correlate the overall movement of a user's hand with the muscle contraction force relative to position.

In at least one instance, the actuator 518 can track the motion of the finger grip lever 516 via the position sensor 520 and match the interaction force via a closed loop, impedance based control. The closed loop, impedance based control can allow the actuator 518 produce a net zero torque, thereby preventing the device from assisting the user with muscle contraction and/or muscle relaxation while still providing dynamic force measurement.

The handgrip myotonia device 500 can detect rotation/pivoting of the finger grip lever 516 and measure the impedance while minimizing interaction force. The handgrip myotonia system (as described in FIG. 1) can measure the muscle relaxation profile by output the torque output of the actuator 518.

The palm rest 514 can be operable to engage with a user's palm during operation while the finger grip lever 516 can be operable to engage with a user's fingers during operation.

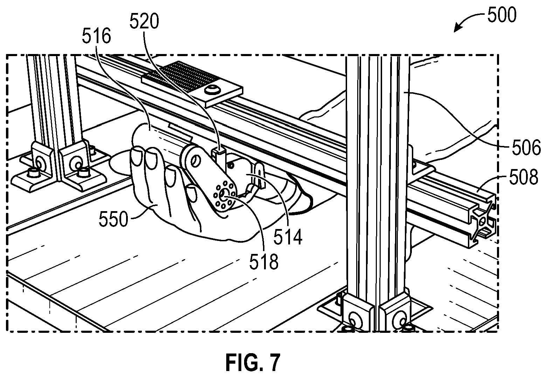

FIG. 7 illustrates a handgrip myotonia device of FIG. 5 during operation. The handgrip myotonia device 500 can operably receive a user's hand 550 by adjacent engagement between the user's palm and the palm rest 514 and the user's fingers and the finger grip lever 516.

As described above with respect to FIG. 1 and FIG. 3, the handgrip myotonia system can generate a cue requesting contraction user's finger muscles. Contraction of the user's finger muscles can rotate and/or pivot the finger grip lever 516 relative to the palm rest 514, and providing the actuator 518 a force measurement (e.g. grip strength). The handgrip myotonia system can subsequently generate a second cue requesting relaxation of user's finger muscles, and allowing the actuator 518 to measure a relaxation profile. In at least one instance, the actuator 518 can detect the relaxation profile as torque output of a servomotor.

In another instance, the position sensor 520 can be set to a predetermined angle of the finger grip lever 516 relative to the palm rest 514. The actuator 518 can be operably engaged to maintain the predetermined angle by generating net torque on the finger grip lever 516 substantially equal to the torque applied by a user's muscle contraction. The net zero torque can allow the finger grip lever 516 to maintain a substantially static position during muscle contraction, while still providing accurate force measurement of muscle contraction.

In yet other instances, during muscle relaxation as a user's finger muscles relax, the actuator 518 can maintain a net zero torque by applying a torque substantially equal to the user's muscle relaxation. Maintaining a net zero torque allows the actuator 516 to track a user's muscle relaxation and/or position without applying a torque to the user's hand to artificial assist muscle relaxation.

While FIGS. 5-7 illustrate the handgrip myotonia device 500 in a substantially palm up orientation, it is within the scope of this disclosure to arrange the handgrip myotonia device 500 in any orientation depending on a user's comfort and/or preference.

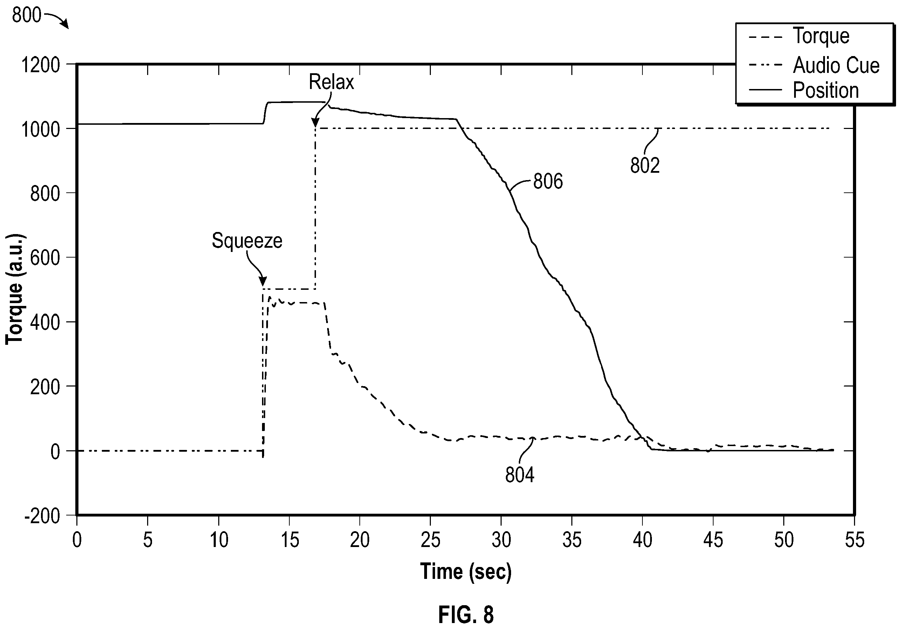

FIG. 8 illustrates a force chart of the handgrip myotonia device of FIG. 5, according to at least one instance of the present disclosure. The force chart 800 can provide data sets recorded by the handgrip myotonia device 500 including position and torque, along with the cues generated by the software suite of the handgrip myotonia system.

The force chart 800 can be a plot of torque (e.g. grip strength), position, and cue over time. The cue 802 can be overlaid on the force chart 800, thereby allowing representation of reaction time of both the user and/or muscles. The measured torque 804 can be plotted as operation of the handgrip myotonia device 500. The measured torque 804 can show the user's generated force in both a muscle contraction and a muscle relaxation situation by overlayment of the cue. The force chart 800 can detail the user's ability to contract muscles upon receipt of the cue and similarly the ability of the user's muscles to relax upon receipt of the second cue after contraction. Thus, delayed muscle relaxation can be identified by the force chart 800 and/or the handgrip myotonia system, thereby allowing medical professionals to potentially identify, characterize, and classify the severity of myotonic dystrophy for further assessing therapeutic response in clinical trials.

Further, the force chart 800 can include measured position 806 over time. The measured position 806 can detail the position of the finger grip lever 516 relative to the palm rest 514. Thus, delayed muscle relaxation can be identified by the force chart 800 and/or the handgrip myotonia system, thereby allowing medical professionals to potentially identify myotonic dystrophy.

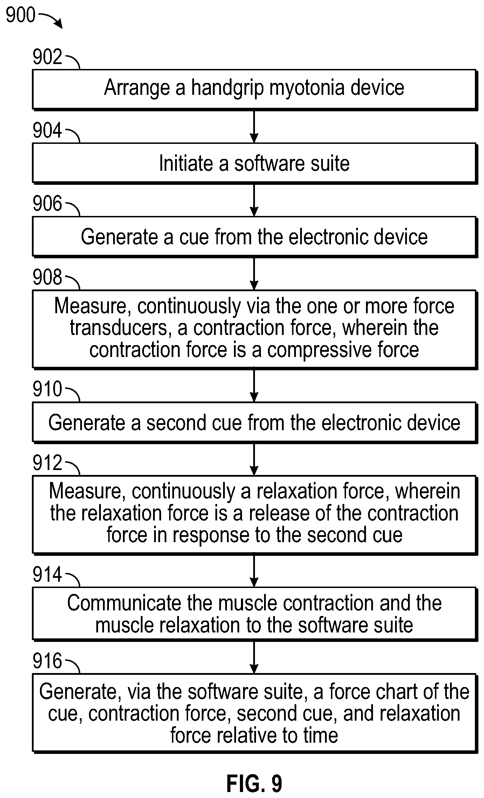

Referring to FIG. 9, a flowchart is presented in accordance with an example method. The example method 900 is provided by way of example, as there is a variety of ways to carry out the method 900. Each block shown in FIG. 9 can represent one or more processes, methods, or subroutines carried out in the example method 900. Furthermore, the illustrated order of blocks is illustrative only and the order of the blocks can change according to the present disclosure. Additional blocks may be added or fewer blocks can be utilized, without deviating from the present disclosure. The example method 900 can begin at block 902.

At block 902, a handgrip myotonia system can be operably arranged to engage a user's hand. The operable arrangement and/or engagement with the user can include adjusting the vertical and/or horizontal positioning of a hand myotonia device relative to the user to ensure comfort and proper alignment for measurement purposes. The method 900 can proceed to block 904.

At block 904, a software suite can be initiated to measure one or more elements of a user's muscles via the handgrip myotonia device. The method 900 can proceed to block 906.

At block 906, the software suite can generate a cue operable to request the user to contract the desired muscle group (e.g. fingers). The cue can be audible, visual, haptic, and/or any other signal operable to communicate to the user the request. The cue can be synchronized as a digital time series with substantially the same sampling rate as other measurements from the handgrip myotonia device, including muscle contraction (block 908) and/or muscle relaxation (block 912) The method 900 can proceed to block 908.

At block 908, the software suite can receive measurements, from one or more force transducers, as the user contracts the desired muscle group (e.g. fingers), thereby compressing one or more force transducers coupled with the handgrip myotonia device. The software suite can synchronize a received muscle contraction measurement with a digital time series in view of the cue. The method 900 can proceed to block 910.

At block 910, the software suite can generate a second cue operable to request the user to relax the desired muscle group (e.g. fingers). The second cue can be audible, visual, haptic, and/or any other signal operable to communicate to the user the request. The method 900 can proceed to block 912.

At block 912, the software suite can receive measurements, from one or more force transducers, as the user relaxes the desired muscle group (e.g. fingers), thereby allowing the one or more force transducers coupled with the handgrip myotonia device to return to a nominal position. The software suite can synchronize a received muscle relaxation measurement with a digital time series in view of the second cue. The method 900 can proceed to block 910.

At block 914, the software suite can generate a display of the measurements for muscle contraction, muscle relaxation in view of time and the cue and the second cue. The software suite can synchronize the cue, muscle contraction, the second cue, and/or the muscle relaxation can be synchronized as a digital time series with substantially the same sampling rate. The display can be a physical print-out, a visual display (e.g. computer screen), and/or combinations thereof. The method 900 can proceed to block 916.

At block 916, the software suite can store the contraction measurements and/or the relaxation measures for each user to a server. The server can be a local hard disk drive (HDD) including a solid state drive (SSD), remote storage, cloud storage, and/or any other data storage medium including combinations thereof.

The invention is defined by the appended claims.

## Claims

1. A handgrip device (500), comprising:
a palm rest (514) operable to engage with a palm of a user;
a grip bar (516) pivotally coupled with the palm rest (514), the grip bar (516) operable to engage with one or more fingers of a user;
one or more force transducers (518) disposed between the palm rest (514) and the grip bar (516), the one or more force transducers (518) operable to measure a compression rate and the return rate of the grip bar (516) relative to the palm rest (514) in response to the grip bar (516) rotating relative to the palm rest (514);
a position sensor (520) coupled with the palm rest (514) and the grip bar (516), the position sensor (520) operable to determine a position of the grip bar (516) relative to the palm rest (514); and
a data connection (512) operable to communicate the compression rate, the return rate and the position of the grip bar (516) relative to the palm rest (514).

2. The handgrip device of claim 1, wherein the one or more force transducers (518) comprises a servomotor.

3. The handgrip device of claim 1, wherein the grip bar (516) is mechanically coupled to the palm rest (514).

4. The handgrip device of claim 1, wherein the grip bar (516) is statically coupled with the palm rest (514).

5. A handgrip system (100) comprising:
a handgrip device (500) according to any preceding claim and an electronic device (102),
wherein the data connection (512) is operable to communicate the compression rate, the return rate and the position of the grip bar (516) relative to the palm rest (514) to the electronic device (102); and
wherein the electronic device (102) has one or more processers (114) and a memory (112) coupled with the one or more processors (114), the one or more processors (114) operable to execute instructions stored on the memory (112) that cause the handgrip system (100) to:
(906) generate a first cue operable to request the user contract muscles;
(908) measure a muscle contraction;
(910) generate a second cue operable to request the user relax muscles;
(912) measure a muscle relaxation; and
(914) generate a force chart including an indication of the first cue, an indication of the second cue, and data corresponding to the muscle contraction and the muscle relaxation.

6. The handgrip system of claim 5, wherein the first cue and the second cue are audio signals.

7. The handgrip system of claim 6, wherein the one or more force transducers (518) are configured to measure a torque profile during relaxation, and wherein the servomotor is configured to maintain a state of minimal interaction or zero impedance between the grip bar and the one or more fingers of the user.

8. A method of handgrip monitoring, the method comprising:
(902) positioning a handgrip device (500) according to claim 1 having a palm rest (514), a grip bar (516) coupled with the palm rest (514), and one or more force transducers (518) disposed between the palm rest (514) and the grip bar (516);
coupling the handgrip device (500) with an electronic device (102) having a software suite (108) installed therein;
(904) starting the software suite (108) to collect data from the handgrip device (500);
(906) generating a first cue from the electronic device (102);
(908) measuring continuously a contraction force, wherein the contraction force is a compressive force in response to the first cue rotating the grip bar (516) relative to the palm rest (514);
communicating the contraction force to the software suite (108);
(910) generating a second cue from the electronic device (102);
(912) measuring continuously a relaxation force, wherein the relaxation force is a release of the contraction force in response to the second cue, thereby allowing the grip bar (516) to rotate and return to a nominal position relative to the palm rest (514);
communicating the relaxation force to the software suite (108); and
(914) generating, by the software suite (108), a force chart including an indication of the first cue, an indication of the second cue, and data corresponding to the contraction force and the relaxation force relative to time;
wherein the grip bar (516) is pivotally coupled with the palm rest (514), and a position sensor (520) is coupled with the palm rest (514) and the grip bar (516), the position sensor (520) operable to determine a position of the grip bar (516) relative to the palm rest (514).

9. The method of claim 8, further comprising maintaining a state of minimal interaction or zero impedance between the grip bar and a user in contact with the grip bar (516) to measure a torque profile during relaxation.

## Patentansprüche

1. Handgriffvorrichtung (500) umfassend
eine zum Zusammenwirken mit einem Handballen eines Benutzers verwendbare Handballenauflage (514),
einen mit der Handballenauflage (514) schwenkbar gekoppelten Griffstab (516), wobei der Griffstab (516) zum Zusammenwirken mit einem Finger bzw. mit mehreren Fingern eines Benutzers ausgelegt ist,
einen oder mehrere zwischen der Handballenauflage (514) und dem Griffstab (516) angeordneten Kraftwandler (518), wobei der eine bzw. die mehreren Kraftwandler (518) zum Messen einer Kompressionsrate und der Rückstellrate des Griffstabs (516) relativ zur Handballenauflage (514) ausgelegt ist,
einen mit der Handballenauflage (514) und dem Griffstab (516) gekoppelten Positionssensor (520), wobei der Positionssensor (520) zum Bestimmen einer Position des Griffstabs (516) relativ zur Handballenauflage (514) aufgrund einer Drehung des Griffstabs (516) relativ zur Handballenauflage (514) ausgelegt ist und
eine zum Übertragen der Kompressionsrate, der Rückstellrate und der Position des Griffstabs (516) relativ zur Handballenauflage (514) ausgelegte Datenverbindung.

2. Handgriffvorrichtung nach Anspruch 1, wobei der eine bzw. die mehreren Kraftumwandler (518) einen Servomotor umfassen.

3. Handgriffvorrichtung nach Anspruch 1, wobei der Griffstab (516) mechanisch mit der Handballenauflage (514) gekoppelt ist.

4. Handgriffvorrichtung nach Anspruch 1, wobei der Griffstab (516) statisch mit der Handballenauflage (514) gekoppelt ist.

5. Handgriffsystem (100) umfassend
eine gemäß einem der vorhergehenden Ansprüche ausgelegte Handgriffvorrichtung sowie ein elektronisches Gerät (102),
wobei die Datenverbindung (512) ausgelegt ist, die Kompressionsrate, die Rückstellrate und die Position des Griffstabs (516) relativ zur Handballenauflage (514) relativ zum elektronischen Gerät (102) zu bestimmen und
wobei das elektronische Gerät (120) einen bzw. mehrere Rechner (114) sowie einen mit dem einen bzw. den mehreren Rechnern (114) gekoppelten Speicher (112) aufweist, wobei der eine bzw. die mehreren Rechner (114) ausgelegt sind, die in dem Speicher (112) gespeicherten Anweisungen auszuführen, die das Handgriffsystem (100) dazu bringen,
(906) einen ersten Hinweis zu erzeugen, der dazu ausgelegt ist, den Benutzer aufzufordern, Muskeln anzuspannen,
(908) eine Muskelspannung zu messen,
(910) einen zweiten Hinweis zu erzeugen, der dazu ausgelegt ist, den Benutzer aufzufordern, Muskeln zu entspannen,
(912) eine Muskelentspannung zu messen und
(914) ein Kraftdiagramm zu erzeugen, das eine Angabe des ersten Hinweises, eine Angabe des zweiten Hinweises und der Muskelanspannung und der Muskelentspnannung entsprechende Daten umfasst.

6. Handgriffsystem nach Anspruch 5, wobei der erste Hinweis und der zweite Hinweis Audiosignale sind.

7. Handgriffsystem nach Anspruch 6, wobei der eine bzw. die mehreren Kraftwandler (518) ausgelegt sind, ein Drehmomentprofil bei der Entspannung zu messen und wobei der Servomotor ausgelegt ist, einen Zustand der minimalen Zusammenwirkung bzw. einer Nullimpedanz zwischen dem Griffstab und dem einen bzw. den mehreren Fingern des Benutzers aufrechtzuerhalten.

8. Verfahren der Handgriffüberwachung umfassend
(902) Positionierung einer Handgriffvorrichtung (502) gemäß Anspruch 1 mit einer Handballenauflage (514), einem Griffstab (516),
der mit der Handballenauflage (514) gekoppelt ist, und einem bzw., mehreren zwischen der Handballenauflage (514) und dem Griffstab (516) angeordneten Kraftwandlern (518),
Koppeln der Handgriffvorrichtung (500) mit einem elektronischen Gerät (102), der ein darauf installiertes Softwarepaket (108) aufweist,
(904) Starten des Softwarepakets (108), um Daten von der Handgriffvorrichtung (500) zu erfassen,
(906) Erzeugen eines ersten Hinweises von dem elektronischen Gerät (102),
(908) kontinuierliches Messen einer Anspannungskraft, wobei die Anspannungskraft eine Druckkraft darstellt, die aufgrund einer Drehung des Griffstabs (516) relativ zur Handballenauflage (514) durch den ersten Hinweis erzeugt wird,
Übertragung der Anspannungskraft an das Softwarepaket (108), (910) Erzeugen eines zweiten Hinweises von dem elektronischen Gerät (102),
(912) kontinuierliches Messen einer Entspannungskraft, wobei die Entspannungskraft eine aufgrund des zweiten Hinweises entstehende Freigabe der Anspannungskraft darstellt, wodurch sich der Griffstab (516) in eine Sollposition relativ zur Handballenauflage (514) drehen und zurückkehren kann,
Übertragung der Entspannungskraft an das Softwarepaket (108) und
(914) Erzeugung durch das Softwarepaket (108) eines Kraftdiagramms, das eine Angabe des ersten Hinweises, eine Angabe des zweiten Hinweises und der Anspannungskraft und der Entspannungskraft entsprechende Daten relativ zur Zeit umfasst,
wobei der Griffstab (516) schwenkbar mit der Handballenauflage (514) gekoppelt ist und ein Positionssensor (520) mit der Handballenauflage (514) und dem Griffstab (516) gekoppelt ist, wobei der Positionssensor (520) zum Bestimmen einer Position des Griffstabs (516) relativ zur Handballenauflage (514) ausgelegt ist.

9. Verfahren nach Anspruch 8, welches weiterhin das Aufrechterhalten einer minimalen Zusammenwirkung bzw. der Nullimpedanz zwischen dem Griffstab und einen den Griffstab (516) anfassenden Benutzer umfasst, um ein Drehmomentprofil bei der Entspannung zu messen.

## Revendications

1. Dispositif de poignée (500), comprenant :
un repose-paume (514) utilisable pour venir en prise avec une paume d'un utilisateur ;
une barre de préhension (516) couplée pivotante au repose-paume (514), la barre de préhension (516) étant utilisable pour venir en prise avec un ou plusieurs doigts d'un utilisateur ;
un ou plusieurs transducteurs de force (518) disposés entre le repose-paume (514) et la barre de préhension (516), le ou les transducteurs de force (518) étant utilisables pour mesurer un taux de compression et le taux de retour de la barre de préhension (516) par rapport au repose-paume (514) en réponse à la rotation de la barre de préhension (516) par rapport au repose-paume (514) ;
un capteur de position (520) couplé au repose-paume (514) et à la barre de préhension (516), le capteur de position (520) étant utilisable pour déterminer une position de la barre de préhension (516) par rapport au repose-paume (514) ; et
une liaison de données (512) utilisable pour communiquer le taux de compression, le taux de retour et la position de la barre de préhension (516) par rapport au repose-paume (514).

2. Dispositif de poignée selon la revendication 1, dans lequel le ou les transducteurs de force (518) comprennent un servomoteur.

3. Dispositif de poignée selon la revendication 1, dans lequel la barre de préhension (516) est couplée mécaniquement au repose-paume (514).

4. Dispositif de poignée selon la revendication 1, dans lequel la barre de préhension (516) est couplée statiquement au repose-paume (514).

5. Système de poignée (100), comprenant :
un dispositif de poignée (500) selon l'une quelconque des revendications précédentes et un dispositif électronique (102),
dans lequel la liaison de données (512) est utilisable pour communiquer le taux de compression, le taux de retour et la position de la barre de préhension (516) par rapport au repose-paume (514) au dispositif électronique (102) ; et
dans lequel le dispositif électronique (102) présente un ou plusieurs processeurs (114) et une mémoire (112) couplée au ou aux processeur(s) (114), le ou les processeurs (114) étant utilisables pour exécuter des instructions enregistrées dans la mémoire (112) qui amènent le système de poignée (100) à :
(906) générer un premier signal utilisable pour demander à l'utilisateur de contracter des muscles ;
(908) mesurer une contraction musculaire ;
(910) générer un second signal utilisable pour demander à l'utilisateur de relâcher des muscles ;
(912) mesurer une relaxation musculaire ; et
(914) générer un diagramme de force comportant une indication du premier signal, une indication du second signal et des données correspondant à la contraction musculaire et à la relaxation musculaire.

6. Système de poignée selon la revendication 5, dans lequel le premier signal et le second signal sont des signaux audios.

7. Système de poignée selon la revendication 6, dans lequel le ou les transducteurs de force (518) sont conçus pour mesurer un profil de couple pendant la relaxation, et dans lequel le servomoteur est conçu pour maintenir un état d'interaction minimale ou d'impédance zéro entre la barre de préhension et le ou les doigts de l'utilisateur.

8. Procédé de surveillance de poignée, le procédé comprenant les étapes consistant à :
(902) positionner un dispositif de poignée (500) selon la revendication 1 présentant un repose-paume (514), une barre de préhension (516) couplée au repose-paume (514) et un ou plusieurs transducteurs de force (518) disposés entre le repose-paume (514) et la barre de préhension (516) ;
coupler le dispositif de poignée (500) à un dispositif électronique (102) présentant une suite logicielle (108) installée sur celui-ci ;
(904) démarrer la suite logicielle (108) pour recueillir des données provenant du dispositif de poignée (500) ;
(906) générer un premier signal au moyen du dispositif électronique (102) ;
(908) mesurer en continu une force de contraction, la force de contraction étant une force de compression en réponse au premier signal faisant tourner la barre de préhension (516) par rapport au repose-paume (514) ;
communiquer la force de contraction à la suite logicielle (108) ;
(910) générer un second signal au moyen du dispositif électronique (102) ;
(912) mesurer en continu une force de relaxation, la force de relaxation étant un relâchement de la force de contraction en réponse au second signal, permettant ainsi à la barre de préhension (516) de tourner et revenir à une position nominale par rapport au repose-paume (514) ;
communiquer la force de relaxation à la suite logicielle (108) ; et
(914) générer, au moyen de la suite logicielle (108), un diagramme de force comportant une indication du premier signal, une indication du second signal et des données correspondant à la force de contraction et à la force de relaxation par rapport au temps ;
dans lequel la barre de préhension (516) est couplée pivotante au repose-paume (514), et un capteur de position (520) est couplé au repose-paume (514) et à la barre de préhension (516), le capteur de position (520) étant utilisable pour déterminer une position de la barre de préhension (516) par rapport au repose-paume (514).

9. Procédé selon la revendication 8, comprenant en outre l'étape consistant à maintenir un état d'interaction minimale ou d'impédance zéro entre la barre de préhension et un utilisateur en contact avec la barre de préhension (516) pour mesurer un profil de couple pendant la relaxation.
